(19) **Europäisches Patentamt / European Patent Office / Office européen des brevets**

(11) **EP 1 980 551 A1**

(12) **EUROPEAN PATENT APPLICATION**
published in accordance with Art. 158(3) EPC

(43) Date of publication:
**15.10.2008 Bulletin 2008/42**

(21) Application number: **07713694.3**

(22) Date of filing: **24.01.2007**

(51) Int Cl.:
**C07C 67/20** (2006.01)       **C07C 69/675** (2006.01)
**C07C 209/14** (2006.01)       **C07C 211/04** (2006.01)

(86) International application number:
**PCT/JP2007/051052**

(87) International publication number:
**WO 2007/088756 (09.08.2007 Gazette 2007/32)**

(84) Designated Contracting States:
**DE**

(30) Priority: **31.01.2006 JP 2006021634**

(71) Applicants:
• **Yamaguchi University**
  **Yamaguchi 753-8511 (JP)**
• **Ube Industries, Ltd.**
  **Ube-shi**
  **Yamaguchi 755-8633 (JP)**

(72) Inventors:
• **KAMIMURA, Akio**
  **Yamaguchi 755-8611 (JP)**
• **SUGIMOTO, Tsunemi**
  **Yamaguchi 755-8633 (JP)**
• **KAISO, Kouji**
  **Yamaguchi 755-8633 (JP)**

(74) Representative: **Bublak, Wolfgang et al**
  **Bardehle, Pagenberg, Dost, Altenburg, Geissler,**
  **Galileiplatz 1**
  **81679 München (DE)**

(54) **PROCESS FOR PRODUCING 6-HYDROXYCAPROIC ESTER AND PROCESS FOR PRODUCING TRIALKYLAMINE**

(57)     A process for producing a 6-hydroxycapoic ester and a process for producing a trialkylamine in both of which nylon-6, ε-caprolactam, which is a monomer therefore, etc. are used as a raw material. The processes are intended to make nylon-6 wastes reusable in various applications without consuming a large quantity of energy.

The process for producing a 6-hydroxycarpoic ester and process for producing a tiralkylamine are **characterized by** reacting amide compounds basically comprising ε-caprolactam with an alcohol in a supercritical state to obtain a 6-hydroxycaproic ester and a trialkylamine.

EP 1 980 551 A1

**Description**

TECHNICAL FIELD

The present invention relates to a process for producing a 6-hydroxycaproic ester useful as an intermediate material in organic syntheses, and a process for producing a trialkylamine useful as an intermediate material for medicines, surfactants, and chemicals in the field of electronics industries.

BACKGROUND ART

Nylon-6 has been used in various applications such as films and engineering plastics. In recent years, nylon-6 has been recycled as emphasis has been placed on reductions in wastes, effective uses of resources and environmental conservation.

Patent Document 1, for example, describes a method of recycling nylon-6, which comprises application of contact with a high-temperature, high-pressure water at 280-450 °C and 100-500 kg/cm$^2$ to depolymerize an ε-caprolactam oligomer into ε-caprolactam.

Patent Document 1: JP 2000-191638A

DISCLOSURE OF THE INVENTION

PROBLEM TO BE SOLVED BY THE INVENTION

The ε-caprolactam obtained from depolymerization of nylon-6 hardly has other uses than the use as a monomer of nylon-6. From the viewpoint of chemical recycling, the reuse of nylon-6 wastes in various applications requires decomposition into hydrogen, carbon monoxide, and methane, which consumes a large quantity of energy as a problem.

On the other hand, a 6-hydroxycaproic ester is useful as an intermediate material in general organic syntheses. In particular, it has been used in various applications such as cation-based coagulants and medicinal/agricultural chemical intermediates, softening agents for synthetic fibers, rust preventing agents, and dispersing agents. The trialkylamine is useful as an intermediate material for medicines, surfactants, and chemicals in the field of electronics industries.

The present invention has an object to provide a process for producing a 6-hydroxycaproic ester and a process for producing a trialkylamine, using nylon-6, or a monomer thereof, ε-caprolactam, as a raw material in order to reuse nylon-6 wastes in various applications without consuming a large quantity of energy.

MEANS TO SOLVE THE PROBLEM

The inventors et al. have eagerly studied to solve the above problem and consequently found that a reaction of an ε-caprolactam-based amide compound, such as nylon-6 and ε-caprolactam, with an alcohol in a supercritical state can produce a 6-hydroxycaproic ester and a trialkylamine at high yields. Namely, the present invention is directed to a process for producing a 6-hydroxycaproic ester, comprising subjecting an ε-caprolactam-based amide compound to a reaction with an alcohol in a supercritical state to obtain a 6-hydroxycaproic ester. The present invention is also directed to a process for producing a trialkylamine, comprising subjecting an ε-caprolactam-based amide compound to a reaction with an alcohol in a supercritical state to obtain a trialkylamine.

EFFECT OF THE INVENTION

As described above, in accordance with the present invention, the reaction of the ε-caprolactam-based amide compound with the alcohol in a supercritical state can provide a process for producing a 6-hydroxycaproic ester and a process for producing a trialkylamine, with a material such as nylon-6 and ε-caprolactam. In the prior chemical recycling, a large quantity of energy is consumed to decompose wastes of nylon-6 and so forth into hydrogen, carbon monoxide, and methane. In contrast, in accordance with the present invention, chemical materials with higher degrees of processability, such as a 6-hydroxycaproic ester and a trialkylamine can be obtained from the wastes of nylon-6 and so forth. Thus, it is possible to achieve chemical recycling of the ε-caprolactam-based amide compound such as nylon-6 with a smaller quantity of energy. THE BEST MODE FOR CARRYING OUT THE INVENTION

In the process of subjecting the ε-caprolactam-based amide compound such as nylon-6 to a reaction with a supercritical alcohol to obtain a 6-hydroxycaproic ester, a trialkylamine can be produced selectively together with the 6-hydroxycaproic ester. In the process for producing a 6-hydroxycaproic ester according to the present invention, the type of the resultant ester can be determined from the type of the alcohol used. For example, if the alcohol is methanol, the resultant 6-hydroxycaproic ester is methyl 6-hydroxycaproate. One of the characteristics of the present invention is that alkyl groups in the trialkylamine obtained in the present invention are all supplied from alkyl groups in the alcohol. Namely, in the process for producing a trialkylamine according to the present invention, the type of the resultant trialkylamine can be determined from the type of the alcohol used. For example, if the alcohol is methanol, the resultant trialkylamine is trimethylamine.

Another characteristic of the present invention is the use of the alcohol in a supercritical state. Preferably, the alcohol available in the process for producing a 6-hydroxycaproic ester and the process for producing a trialkylamine according to the present invention is a primary alcohol. Examples include methanol, ethanol, n-propanol, n-butanol, n-pentanol, and n-hexanol.

The above alcohol can be brought into a supercritical state by heating and pressurizing, or heating in a hermetically sealed state. The process for producing a 6-hydroxycaproic ester according to the present invention may comprise mixing an ε-caprolactam-based amide compound with an alcohol, and then bringing the alcohol into a supercritical state by heating. Alternatively, it may comprise adding an alcohol in a supercritical state to the ε-caprolactam-based amide compound. The critical temperatures and critical pressure of the major alcohol are as shown in Table 1.

[Table 1]

|  | Critical Temperature (°C) | Critical Pressure (MPaG) |
|---|---|---|
| Methanol | 239 | 8.1 |
| Ethanol | 243 | 6.4 |
| n-Propanol | 264 | 5.2 |
| n-Butanol | 290 | 4.4 |

Examples of the ε-caprolactam-based amide compound used as the material in the process for producing a 6-hydroxycaproic ester and the process for producing a trialkylamine according to the present invention include an ε-caprolactam; a methylated ε-caprolactam derivative such as N-methyl caprolactam; a polymer of ε-caprolactam, that is, nylon-6; and a low polymer of ε-caprolactam, that is, an oligomer of ε-caprolactam. More specific examples include wastes of nylon-6 fiber carpets, and out-of-spec products including an oligomer produced during polymerization of caprolactam to produce nylon-6.

In the process for producing a 6-hydroxycaproic ester and the process for producing a trialkylamine according to the present invention, the reaction temperature is 250-400 °C, preferably 330-370 °C. In addition, the reaction pressure is 15-40 MPaG (G denotes gauge pressure) , preferably 27-36 MPaG. Further, the reaction time is 0.5-6 hours, preferably 1-3 hours. The weight of the ε-caprolactam-based amide compound is more than 0 and not more than 50 wt. %, preferably more than 0 and not more than 10 wt. % on the basis of the total weight of the ε-caprolactam-based amide compound and the alcohol.

The 6-hydroxycaproic ester-containing reaction solution obtained in accordance with the present invention may be subjected to flash distillation to separate/remove the trialkylamine and alcohol. If a high-purity 6-hydroxycaproic ester is desired, the residue after separation/removal of the trialkylamine and alcohol is purified through vaccum distillation. The trialkylamine separated/removed together with the alcohol can be purified through normal-pressure distillation or vaccum distillation.

EXAMPLE 1

Examples of the process for producing a methyl 6-hydroxycaproate according to the present invention will now be described below. First, in Example 1, a 10mL-volume, SUS 316 (stainless steel) reaction tube was provided, into which 0.3 g of nylon-6 (Nylon Chip 1022B from Ube Industries) and 4 g of methanol (with a water content of 1600 ppm) were supplied. The air inside the dead space in the reaction tube was replaced with argon and sealed. The sealed reaction tube was left stationary for one hour (under pressure of 20.3 MPaG) in an electrical furnace heated up to 300 °C. Thereafter, the reaction tube was extracted from the electrical furnace. The reaction tube was then immersed into water and cooled down to the room temperature.

EXAMPLE 2

In Example 2, a methyl 6-hydroxycaproate was produced in a similar manner as Example 1 except that the reaction time was changed to 2 hours (under pressure of 20.3 MPaG).

EXAMPLE 3

In Example 3, a methyl 6-hydroxycaproate was produced in a similar manner as Example 1 except that the reaction time was changed to 3 hours (under pressure of 20.3 MPaG).

EXAMPLE 4

In Example 4, a methyl 6-hydroxycaproate was produced in a similar manner as Example 1 except that the reaction time was changed to 4 hours (under pressure of 20.3 MPaG).

EXAMPLE 5

In Example 5, a methyl 6-hydroxycaproate was produced in a similar manner as Example 1 except that the reaction time was changed to 5 hours (under pressure of 20.3 MPaG).

EXAMPLE 6

In Example 6, a methyl 6-hydroxycaproate was produced in a similar manner as Example 1 except that the reaction time was changed to 6 hours (under pressure of 20.3 MPaG).

EXAMPLE 7

In Example 7, a methyl 6-hydroxycaproate was produced in a similar manner as Example 1 except that the reaction temperature was changed to 330 °C (under pressure of 27.0 MPaG).

EXAMPLE 8

In Example 8, a methyl 6-hydroxycaproate was produced in a similar manner as Example 7 except that the reaction time was changed to 2 hours (under pressure of 27.0 MPaG).

EXAMPLE 9

In Example 9, a methyl 6-hydroxycaproate was produced in a similar manner as Example 7 except that the reaction time was changed to 3 hours (under pressure of 27.0 MPaG).

EXAMPLE 10

In Example 10, a methyl 6-hydroxycaproate was produced in a similar manner as Example 7 except that the reaction time was changed to 4 hours (under pressure of 27.0 MPaG).

EXAMPLE 11

In Example 11, a methyl 6-hydroxycaproate was produced in a similar manner as Example 7 except that the reaction time was changed to 5 hours (under pressure of 27.0 MPaG).

EXAMPLE 12

In Example 12, a methyl 6-hydroxycaproate was produced in a similar manner as Example 7 except that the reaction time was changed to 6 hours (under pressure of 27.0 MPaG).

EXAMPLE 13

In Example 13, a methyl 6-hydroxycaproate was produced in a similar manner as Example 1 except that the reaction temperature was changed to 350 °C (under pressure of 31.0 MPaG).

EXAMPLE 14

In Example 14, a methyl 6-hydroxycaproate was produced in a similar manner as Example 13 except that the reaction time was changed to 2 hours (under pressure of 31.0 MPaG).

EXAMPLE 15

In Example 15, a methyl 6-hydroxycaproate was produced in a similarmanner as Example 13 except that the reaction time was changed to 3 hours (under pressure of 31.0 MPaG).

EXAMPLE 16

In Example 16, a methyl 6-hydroxycaproate was produced in a similar manner as Example 1 except that the reaction temperature was changed to 370 °C (under pressure of 35.3 MPaG).

EXAMPLE 17

In Example 17, a methyl 6-hydroxycaproate was produced in a similar manner as Example 16 except that the reaction time was changed to 2 hours (under pressure of 35.3 MPaG).

EXAMPLE 18

In Example 18, a methyl 6-hydroxycaproate was produced in a similarmanner as Example 16 except that the reaction time was changed to 3 hours (under pressure of 35.3 MPaG).

Next, the reaction mixtures in Examples 1-18 were extracted from the autoclave to quantify ε-caprolactam, N-methyl caprolactam and methyl 6-hydroxycaproate. The quantification of ε-caprolactam, N-methyl caprolactam and methyl 6-hydroxycaproate was performed with a gas chromatography (GC-14B from Shimadzu Corporation). The yields of reaction products such as ε-caprolactam, N-methyl caprolactam and methyl 6-hydroxycaproate were calculated based on the following Equation 1. These results are shown in Table 2.

[Equation 1]

Yield of Reaction Product (%) =
[(Reaction Product in Reaction Mixture (wt. %)) × (Total Mass of Nylon-6, Solvent and Catalyst before Reaction (g)]/(Mass of Nylon-6 before Reaction (g)) × 100

If no catalyst is used, then Mass of Catalyst is made zero (g).

[Table 2]

|  | ε-caprolactam | N-methyl caprolactam | methyl 6-hydroxycaproate |
|---|---|---|---|
| Example 1 | 7.7 | 0.8 | 0.5 |
| Example 2 | 20.2 | 3.7 | 2.6 |
| Example 3 | 28.8 | 7.3 | 7.7 |
| Example 4 | 29.0 | 17.0 | 22.7 |
| Example 5 | 18.9 | 9.7 | 11.7 |
| Example 6 | 15.8 | 6.5 | 12.1 |
| Example 7 | 25.7 | 7.3 | 5.8 |
| Example 8 | 27.2 | 17.4 | 30.4 |

(continued)

|  | ε-caprolactam | N-methyl caprolactam | methyl 6-hydroxycaproate |
|---|---|---|---|
| Example 9 | 14.4 | 17.9 | 40.4 |
| Example 10 | 0.8 | 3.7 | 40.7 |
| Example 11 | 0.6 | 2.8 | 49.1 |
| Example 12 | 0.3 | 2.8 | 64.7 |
| Example 13 | 22.0 | 15.0 | 25.5 |
| Example 14 | 13.0 | 19.3 | 43.6 |
| Example 15 | 2.9 | 8.9 | 69.1 |
| Example 16 | 9.0 | 17.5 | 59.7 |
| Example 17 | 1.5 | 1.9 | 59.9 |
| Example 18 | 0.7 | 1.0 | 70.7 |
|  |  |  | (%) |

As shown in Table 2, it can be confirmed that the reaction of nylon-6 with the alcohol in a supercritical state can produce methyl 6-hydroxycaproate.

As for Example 18, the gas chromatography is used to quantify trimethylamine and the yield of the produced trimethylamine is calculated 23.0 % based on Equation 1. The gas chromatography confirmed the production of trimethylamine.

EXAMPLE 19

Next, in Example 19, a 10mL-volume, SUS 316 (stainless steel) reaction tube was provided, into which 0.3 g of ε-caprolactam (from Ube Industries) and 4 g of methanol (with a water content of 1600 ppm) were supplied. The air inside the dead space in the reaction tube was replaced with argon and sealed. The sealed reaction tube was left stationary for one hour (under pressure of 27.0 MPaG) in an electrical furnace heated up to 330 °C. Thereafter, the reaction tube was extracted from the electrical furnace. The reaction tube was then immersed into water and cooled down to the room temperature.

EXAMPLE 20

In Example 20, a methyl 6-hydroxycaproate was produced in a similar manner as Example 19 except that the reaction time was changed to 2 hours (under pressure of 27.0 MPaG).

EXAMPLE 21

In Example 21, a methyl 6-hydroxycaproate was produced in a similar manner as Example 19 except that the reaction time was changed to 3 hours (under pressure of 27.0 MPaG).

EXAMPLE 22

In Example 22, a methyl 6-hydroxycaproate was produced in a similar manner as Example 19 except that the react ion time was changed to 4 hours (under pressure of 27.0 MPaG).

EXAMPLE 23

In Example 23, a methyl 6-hydroxycaproate was produced in a similar manner as Example 19 except that the reaction time was changed to 5 hours (under pressure of 27.0 MPaG).

EXAMPLE 24

In Example 24, a methyl 6-hydroxycaproate was produced in a similar manner as Example 19 except that the reaction time was changed to 6 hours (under pressure of 27.0 MPaG).

Next, the reaction mixtures in Examples 19-24 were extracted from the autoclave to quantify ε-caprolactam, N-methyl caprolactam and methyl 6-hydroxycaproate. The quantification of ε-caprolactam, N-methyl caprolactam and methyl 6-hydroxycaproate was performed with a gas chromatography (GC-14B from Shimadzu Corporation). The yields of non-reacted ε-caprolactam and of produced N-methyl caprolactam and methyl 6-hydroxycaproate were calculated based on the above Equation 1. These results are shown in Table 3.

[Table 3]

|  | ε-caprolactam | N-methyl caprolactam | methyl 6-hydroxycaproate |
|---|---|---|---|
| Example 19 | 75.2 | 12.9 | 0.9 |
| Example 20 | 39.2 | 21.8 | 4.6 |
| Example 21 | 14.9 | 25.7 | 33.8 |
| Example 22 | 3.4 | 14.7 | 50.2 |
| Example 23 | 0.4 | 2.2 | 55.2 |
| Example 24 | 0.3 | 3.4 | 59.0 |
|  |  |  | (%) |

As shown in Table 3, it can be confirmed that the reaction of ε-caprolactam with the alcohol in a supercritical state can produce methyl 6-hydroxycaproate.

**Claims**

1.  A process for producing a 6-hydroxycaproic ester, comprising subjecting an ε-caprolactam-based amide compound to a reaction with an alcohol in a supercritical state to obtain a 6-hydroxycaproic ester.

2.  The process for producing a 6-hydroxycaproic ester according to claim 1, wherein the reaction is performed at a temperature of 330-370 °C.

3.  The process for producing a 6-hydroxycaproic ester according to claim 1 or 2, wherein the alcohol comprises methanol.

4.  The process for producing a 6-hydroxycaproic ester according to any one of claims 1-3, wherein the reaction with an alcohol in a supercritical state is also used to obtain a trialkylamine together.

5.  A process for producing a trialkylamine, comprising subjecting an ε-caprolactam-based amide compound to a reaction with an alcohol in a supercritical state to obtain a trialkylamine.

6.  The process for producing a trialkylamine according to claim 5, wherein the reaction is performed at a temperature of 330-370 °C.

7.  The process for producing a trialkylamine according to claim 5 or 6, wherein the alcohol comprises methanol.

<table>
<tr><td colspan="2">**INTERNATIONAL SEARCH REPORT**</td><td>International application No.<br>PCT/JP2007/051052</td></tr>
</table>

A.  CLASSIFICATION OF SUBJECT MATTER
*C07C67/20*(2006.01)i, *C07C69/675*(2006.01)i, *C07C209/14*(2006.01)i,
*C07C211/04*(2006.01)i

According to International Patent Classification (IPC) or to both national classification and IPC

B.  FIELDS SEARCHED

Minimum documentation searched (classification system followed by classification symbols)
C07C67/20, C07C69/675, C07C209/14, C07C211/04

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)
CAplus(STN), REGISTRY(STN)

C.  DOCUMENTS CONSIDERED TO BE RELEVANT

| Category* | Citation of document, with indication, where appropriate, of the relevant passages | Relevant to claim No. |
|---|---|---|
| X<br>A | JP 2002-148253 A  (Sumitomo Chemical Co., Ltd.,<br>Sumika Chemical Analysis Service, Ltd.),<br>22 May, 2002 (22.05.02),<br>(Family: none) | 1-3<br>4-7 |
| A | JP 11-343338 A  (Toray Industries, Inc.),<br>14 December, 1999 (14.12.99),<br>(Family: none) | 1-7 |
| A | JP 2000-191638 A  (Ube Industries, Ltd.,<br>Kobe Steel, Ltd.),<br>11 July, 2000 (11.07.00),<br>(Family: none) | 1-7 |

☐  Further documents are listed in the continuation of Box C.          ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>28 February, 2007 (28.02.07) | Date of mailing of the international search report<br>06 March, 2007 (06.03.07) |
|---|---|
| Name and mailing address of the ISA/<br>Japanese Patent Office | Authorized officer |
| Facsimile No. | Telephone No. |

Form PCT/ISA/210 (second sheet) (April 2005)

**EP 1 980 551 A1**

## REFERENCES CITED IN THE DESCRIPTION

### Patent documents cited in the description

• JP 2000191638 A